# EUROPÄISCHE PATENTSCHRIFT

(11) **EP 1 927 322 B1**
(45) Veröffentlichungstag und Bekanntmachung des Hinweises auf die Patenterteilung: **02.06.2010**
(21) Anmeldenummer: 06125231.8
(22) Anmeldetag: 01.12.2006
(51) Int. Cl.: A61B 17/86

(54) **Vorrichtung zum Positionieren von Röhrenknochen**
Device for positioning tubular bones
Dispositif de positionnement des os tubulaires

(43) Veröffentlichungstag der Anmeldung: 04.06.2008
(73) Patentinhaber: Wright Medical Technology, Inc., Arlington, Tennessee 38002 (US)
(72) Erfinder: Shibuya,Naohiro,DPM, San Antonio, TX 78238 (US)
(74) Vertreter: Rupprecht, Kay

(56) Entgegenhaltungen:
- FR-A1- 2 808 182
- US-A- 5 919 193
- US-A1- 2003 045 881

## Beschreibung

Die vorliegende Erfindung betrifft eine Vorrichtung zum Positionieren der Röhrenknochen benachbarter erster und zweiter Zehenglieder eines Patienten auf der Zehenlängsachse, wobei die Vorrichtung eine zweigängige Knochenschraube mit einem im wesentlichen zylindrischen proximalen Endabschnitt, einem im wesentlichen zylindrischen distalen Endabschnitt und mit einem zwischen dem proximalen und distalen Endabschnitt angeordneten und im wesentlichen zylindrischen Mittenabschnitt aufweist, wobei zwischen dem proximalen Endabschnitt und dem Mittenabschnitt der Knochenschraube ein proximaler Gewindeabschnitt mit einem ersten Gewinde und zwischen dem distalen Endabschnitt und dem Mittenabschnitt der Knochenschraube ein distaler Gewindeabschnitt mit einem zweiten Gewinde vorgesehen sind, und wobei das erste Gewinde eine Gewindesteigung aufweist, die kleiner als die Gewindesteigung des zweiten Gewindes ist. Es wird auch die Verwendung einer derartigen Vorrichtung offenbart als Einrichtung zum Positionieren der Röhrenknochen von zwei benachbarten Zehengliedern eines Patienten in einer Korrekturstellung auf einer Zehenlängsachse und zum gleichzeitigen Aufbringen einer in Richtung der Zehenlängsachse auf die Röhrenknochen der benachbarten Zehenglieder wirkenden einstellbaren Druckspannung bei der operativen Behandlung eines Hammerzehs oder eines Krallenzehs, oder bei der operativen Behandlung eines Hallux Valgus oder eines Hallux Rigidus, bei welcher jeweils eine resektiosarthroplastische Operationstechnik zum Einsatz kommt.

Die Verwendung von zweigängigen Knochenschrauben ist aus der Medizintechnik im Zusammenhang mit der Behandlung von Kahnbeinfrakturen (Skaphoidfrakturen) bekannt. Beispielsweise werden in den Druckschriften US 4,175,555 und US 5,019,079 jeweils ein kanüliertes Schraubsystem zum perkutanen Verschrauben von Skaphoidfrakturen beschrieben, wobei dieses bekannte Schraubsystem die Möglichkeit einer Kompression von Frakturlinien bietet.

Die aus dem Stand der Technik bekannte Kompressionsschraube erfordert eine Eröffnung der Handgelenkkapsel, gegebenenfalls eine Reposition und anschließend die Kompression mit einem Zielgerät, dem so genannten "Jig". Das Schraubsystem dient dabei zum Stabilisieren des mit einer Fraktur versehenen Kahnbeinknochens, wobei die Schraube vollständig im Knochen versenkt und nicht wieder entfernt werden kann. Demnach dient das aus der Handchirurgie bekannte Schraubsystem als Kompressionsschraube zur Knochenbruchheilung, wobei zwei Knochenfragmente durch das Einbringen des Schraubsystems dauerhaft zusammengepresst werden.

Bei dieser aus dem Stand der Technik bekannten Kompressionsschraube handelt es sich allerdings um eine Knochenschraube, die speziell für die nicht-konservative Therapie von Skaphoidfrakturen in der Handchirurgie entwickelt wurde, wobei die Knochenschraube ausgelegt ist, die zu verbindenden Knochenbruchstücke des Kahnbeins fest aneinander zu drücken.

Die Verwendung einer derartigen Kompressionsschraube zum Positionieren der Röhrenknochen benachbarter erster und zweiter Zehenglieder eines Patienten in einer Korrekturstellung auf einer Zehenlängsachse und zum gleichzeitigen Aufbringen einer in Richtung der Zehenlängsachse auf die Röhrenknochen der benachbarten Zehenglieder wirkenden, genau einstellbaren Druckspannung bei der operativen Behandlung von beispielsweise einem Hammerzeh oder einem Krallenzeh, ist derzeit nicht möglich, da die aus dem Gebiet der Handchirurgie bekannte Kompressionsschraube in struktureller Hinsicht nicht ausgelegt ist, einerseits zwei Knochen, die üblicherweise über ein Gelenk miteinander verbunden sind, nach einem resektiosarthroplastischen Eingriff in einer Korrekturstellung zu positionieren, während andererseits gleichzeitig auf den Knochen eine genau einzustellende leichte Spannung auszuüben ist.

Bei der operativen Behandlung von beispielsweise einem Hammerzeh oder einem Krallenzeh sind allerdings die möglichst genaue Positionierung der Röhrenknochen benachbarter erster und zweiter Zehenglieder in einer Korrekturstellung auf der Zehenlängsachse und das gleichzeitige Aufbringen einer in Richtung der Zehenlängsachse auf die Röhrenknochen wirkenden, genau dosierbaren Druckspannung wesentliche Maßnahmen, die im Hinblick auf eine erfolgreiche Rekonstruktion der behandelten Zehe und im Hinblick auf die Wiederherstellung eines voll belastbaren Fußes ausschlaggebend sind.

Hammerzehen und Krallenzehen sind Zehenfehlstellungen, die oft zusammen mit einem Hallux Valgus und einem Spreizfuß auftreten. Bei einem Hammerzeh handelt es sich um eine isolierte maximale Beugung der betroffenen Zehe im Endgelenk, während sich Krallenzehen durch eine Überstreckung des Grundgelenkes bei gebeugtem Mittel- und Zehenendgelenk auszeichnen. Da selbst recht ausgeprägte Fehlstellungen häufig sehr schmerzarm sind, die die Lebensqualität des Patienten nicht wesentlich einschränken, ist in vielen Fällen eine operative Behandlung bei einer Hammerzehen-Fehlstellung oder einer Krallenzehen-Fehlstellung aus rein kosmetischen Gründen erforderlich.

Andererseits kann die mit einem Hammerzeh bzw. Krallenzeh einhergehende Fußverformung auch dazu führen, dass beim Patienten die fehlstehenden Zehen am Schuh drücken, wodurch mechanische Reizzustände mit Schwielenbildung (Hühneraugen) und oft auch Entzündungen und chronische Schmerzen entstehen können.

Das Ziel der operativen Behandlung einer derartigen Zehenfehlstellung ist die Korrektur der Fehlstellung und der gegebenenfalls bereits eingetretenen Versteifung, sowie die Entlastung der passiven Sehnenspannung durch eine Verkürzung der Knochenstrecke. Hierbei wird beispielsweise mit einer sogenannten "Operation nach Hohmann" ein Teil des Zehenknochens entfernt. Bei dieser Operation wird üblicherweise bei dem nach oben vorspringenden Köpfchen des Grundzehenknochens eine Resektion (Entfernung) an der Stelle durchgeführt, an der das Hühnerauge bzw. die Schwiele sitzt. Im einzelnen wird insbesondere bei Vorliegen von rigiden Krallen- oder Hammerzehenfehlstellungen während des resektionsarthroplastischen Eingriffes häufig zunächst das Köpfchen des Grundgliedes entfernt, wobei anschließend eine Ausdehnung der verkürzten Beugesehne durch manuelle Korrektur stattfindet.

Unabhängig von der bei der Rekonstruktion der behandelten Zehe zum Einsatz kommenden Operationstechnik ist es grundsätzlich erforderlich, nach der Stellungskorrektur der Zehenknochen, die Röhrenknochen der benachbarten Zehenglieder des Patienten zu stabilisieren. Üblicherweise wird hierzu als eine innere Schienung zur Sicherstellung des Korrekturergebnisses ein dünner Draht, wie beispielsweise ein Bohrdraht oder ein sogenannter "Kirschnerdraht", im Verlauf der Zehenlängsachse eingebracht.

Der dabei zum Einsatz kommende Draht dient allerdings nicht nur als eine innere Schienung zur Sicherstellung des Korrekturergebnisses, sondern auch dazu, die behandelte Zehe unter einer während des Operationseingriffes einzustellenden leichten Spannung in Korrekturstellung zu halten. Diese Maßnahme ist für eine Zeitdauer von etwa 2 Wochen erforderlich, innerhalb welcher die während des operativen Eingriffes absichtlich abgelöste Abduktorensehne an der medialen Seite der Grundphalanx wieder angewachsen ist. Nach dieser Zeit muss der Draht wieder aus den Zehengliedern entfernt werden.

Demnach dient derzeit der bei der operativen Behandlung einer Zehenfehlstellung zum Einsatz kommende Draht zur Sicherung des Korrekturergebnisses und insbesondere zum Halten der Zehen nach dessen bei der operativen Rekonstruktion erfolgten korrekten Einstellung.

Da bei jeder Rekonstruktion die Wiederherstellung eines voll belastbaren Fußes angestrebt wird, ist die korrekte Einstellung der behandelten Zehe bekannterweise der schwierigste Teil der Operation und erfordert eine große und langjährige Erfahrung des behandelnden Chirurgen. Dies gilt insbesondere für das Einbringen des Drahtes in die zu schienenden Röhrenknochen der benachbarten Zehenglieder sowie für das Aufbringen der optimalen (leichten) Spannung, unter welcher die benachbarten Zehenglieder der behandelten Zehe gehalten werden. Insbesondere ist es dabei erforderlich, den Abstand der benachbarten Zehenglieder der behandelten Zehe möglichst genau einzustellen, so dass die behandelte Zehe in ihrer natürlichen (korrigierten) Stellung fixiert wird.

Der vorliegenden Erfindung liegt nun die Problemstellung zugrunde, dass das Einbringen des Drahtes zum Fixieren des Operationsergebnisses und zum Halten der behandelten Zehe nach erfolgter Stellungskorrektur bei einer operativen Rekonstruktion der Zehe die langjährige Erfahrung des Chirurgen erfordert, und insbesondere relativ zeitaufwendig ist, da mit Hilfe des Drahtes auch die leichte Spannung genau einzustellen ist, unter welcher die benachbarten Zehenglieder der behandelten Zehe gehalten werden müssen. Aus diesem Grund ist die operative Behandlung eines Hammerzehs oder eines Krallenzehs auch relativ kostenaufwendig. Andererseits besteht die Gefahr, dass bei einem nicht optimalen Einbringen des Drahtes die Korrekturstellung der behandelten Zehe nicht ausreichend fixiert wird, infolgedessen die behandelte Zehe selbst nach dessen Rekonstruktion eine Fehlstellung beibehält und die korrekte Einstellung des Fußes nicht erreicht wird.

Ausgehend von dieser Problemstellung liegt der vorliegenden Erfindung demnach die Aufgabe zugrunde, eine medizinische Vorrichtung zum Positionieren der Röhrenknochen benachbarter erster und zweiter Zehenglieder eines Patienten in einer bei einem operativen Eingriff eingestellten Korrekturstellung auf der Zehenlängsachse anzugeben, wobei diese medizinische Vorrichtung während des Operationseingriffes ohne größere Schwierigkeiten und Vorkenntnisse des verantwortlichen Chirurgen in einer behandelten Zehe des Patienten implantierbar ist, so dass ohne größeren Aufwand die korrekte Einstellung der behandelten Zehe auch bei komplexen Rekonstruktionen möglich ist. Insbesondere soll die medizinische Vorrichtung bei einem Routineeingriff in der Zehe des Patienten einsetzbar sein, wobei ohne größeren Aufwand nicht nur die Fixierung der benachbarten Zehenglieder der behandelten Zehe, sondern auch das Einstellen der optimalen, auf die benachbarten Zehenglieder auszuübenden (leichten) Spannung ermöglicht wird.

Diese Aufgabe wird erfindungsgemäß mit einer medizinischen Vorrichtung gelöst, die eine zweigängige Knochenschraube mit einem im wesentlichen zylindrischen proximalen Endabschnitt, einem im wesentlichen zylindrischen distalen Endabschnitt und einem zwischen dem proximalen und distalen Endabschnitt angeordneten und im wesentlichen zylindrischen Mittenabschnitt aufweist, wobei zwischen dem proximalen Endabschnitt und dem Mittenabschnitt der Knochenschraube ein proximaler Gewindeabschnitt mit einem ersten Gewinde und zwischen dem distalen Endabschnitt und dem Mittenabschnitt der Knochenschraube ein distaler Gewindeabschnitt mit einem zweiten Gewinde vorgesehen sind, und wobei das erste Gewinde eine Gewindesteigung aufweist, die kleiner als die Gewindesteigung des zweiten Gewindes ist, und wobei der Nenndurchmesser des ersten Gewindes größer als der Durchmesser des proximalen Endabschnittes und der Nenndurchmesser des zweiten Gewindes größer als der Durchmesser des distalen Endabschnittes und gleich groß wie oder kleiner als der Durchmesser des proximalen Endabschnittes ist, und wobei der proximale Endabschnitt der Knochenschraube wenigstens gleich lang wie der Mittenabschnitt ist. Eine Schraube die diese Merkmale aufweist ist aus der Patentanmeldung FR-A-2 808 182 bekannt.

Unter dem hierin verwendeten Begriff "distaler Endabschnitt der Knochenschraube" ist der vordere Endabschnitt der Knochenschraube zu verstehen, welcher in Einschraubrichtung der Knochenschraube liegt. Demgemäß ist unter dem Begriff "proximaler Endabschnitt" der dem distalen Endabschnitt der Knochenschraube gegenüberliegende Endabschnitt der Knaochenschraube zu verstehen. Bei den übrigen Verwendungen der Begriffe "proximal" und "distal" gilt eine entsprechende Definition.

Bei der erfindungsgemäßen medizinischen Vorrichtung kommt somit eine zweigängige Knochenschraube zum Einsatz, bei welcher durch das Vorsehen des proximalen Endabschnittes, des distalen Endabschnittes und des dazwischen angeordneten Mittenabschnittes die Verwendung dieser Knochenschraube als Einrichtung zum Positionieren der Röhrenknochen benachbarter Zehenglieder einer behandelten Zehe in Korrekturstellung auf der Zehenlängsachse möglich ist. Hierzu dienen insbesondere der distale und proximale Endabschnitt der Knochenschraube zur Festlegung der Korrekturstellung der entsprechenden Röhrenknochen auf der Zehenlängsachse. Dabei liegt im implantierten Zustand der Knochenschraube der distale Endabschnitt und der distale Gewindeabschnitt der Knochenschraube im Inneren des Röhrenknochen des ersten Zehengliedes, während der proximale Endabschnitt und der proximale Gewindeabschnitt der Knochenschraube durch das Innere des Röhrenknochens des zweiten Zehengliedes läuft. Die jeweiligen Gewinde des proximalen und distalen Gewindeabschnittes stehen dabei in einem lösbaren Eingriff mit den entsprechenden Röhrenknochen.

Indem für den Nenndurchmesser des ersten Gewindes ein Wert gewählt wird, der größer als der Durchmesser des proximalen Endabschnittes ist, und indem zusätzlich für den Nenndurchmesser des zweiten Gewindes ein Wert gewählt wird, der größer als der Durchmesser des distalen Endabschnittes und gleich groß wie oder kleiner als der Durchmesser des proximalen Endabschnittes der Knochenschraube ist, kann erreicht werden, dass vor dem Einbringen der Knochenschraube in die zu fixierende Zehe des Patienten ein Bohrloch in die Röhrenknochen der benachbarten ersten und zweiten Zehenglieder der behandelten Zehe eingebracht werden kann, welches vorzugsweise längs der Zehenlängsachse verläuft, wobei anschließend beim Einbringen (Einschrauben) der Knochenschraube diese Bohrung mit dem ersten und zweiten Gewinde der Knochenschraube überbohrt wird. Dadurch können die benachbarten Röhrenknochen der ersten und zweiten Zehenglieder der behandelten Zehe mit Hilfe der Knochenschraube in einer idealen Ausrichtung zueinander auf der Zehenlängsachse positioniert werden.

Da erfindungsgemäß das erste Gewinde eine Gewindesteigung aufweist, die kleiner als die Gewindesteigung des zweiten Gewindes ist, werden durch eine Drehung der bereits eingesetzten Knochenschraube die beiden benachbarten Zehenglieder der behandelten Zehe relativ zueinander aufeinander zubewegt, da mit dem ersten Gewinde des proximalen Gewindeabschnittes auf den Röhrenknochen des zugehörigen Zehengliedes in Zehenlängsachse (je nach Drehung der Knochenschraube) eine Zug- bzw. Druckspannung ausgeübt wird, deren Betrag verschieden von der Zug- bzw. Druckspannung ist, die über das zweite Gewinde des distalen Gewindeabschnittes auf den Röhrenknochen des benachbarten Zehengliedes ausgeübt wird. Dadurch, dass zwischen dem proximalen und distalen Gewindeabschnitt ein Mittenabschnitt mit einem definierten Abstand vorgesehen ist, infolgedessen das erste und zweite Gewinde der Knochenschraube voneinander definiert beabstandet sind, kann durch eine entsprechende Drehung der Knochenschraube der Abstand zwischen dem ersten und dem benachbarten zweiten Zehenglied der behandelten Zehe sehr genau eingestellt werden.

Somit ist die medizinische Vorrichtung gemäß der vorliegenden Erfindung, welche die zweigängige Knochenschraube aufweist, einerseits zum Positionieren der Röhrenknochen benachbarter Zehenglieder einer behandelten Zehe in der Korrekturstellung auf der Zehenlängsachse und andererseits zum gleichzeitigen (genauen) Einstellen eines vorgebbaren Abstandes zwischen den benachbarten Zehengliedern geeignet.

Insbesondere kann, indem der Abstand zwischen den benachbarten Zehengliedern der behandelten Zehe entsprechend verringert wird, auf die Röhrenknochen der benachbarten Zehenglieder eine genau einstellbare (leichte) Spannung in Richtung der Zehenlängsachse aufgebracht werden, was für eine korrekte Einstellung der behandelten Zehe, insbesondere bei komplexen Rekonstruktionen, erforderlich ist.

Vorteilhafte Ausführungsformen der erfindungsgemäßen Vorrichtung sind in den Unteransprüchen angegeben.

In einer bevorzugten Realisierung der bei der erfindungsgemäßen Vorrichtung zum Einsatz kommenden Knochenschraube ist vorgesehen, dass der proximale Endabschnitt, der distale Endabschnitt und der Mittenabschnitt zwischen dem proximalen und distalen Endabschnitt der Knochenschraube jeweils als gewindefreie Kreiszylinder ausgebildet sind. Dies ist insbesondere im Hinblick auf das mit der medizinischen Vorrichtung bewirkte Aufbringen einer leichten, genau einstellbaren Spannung auf die benachbarten Zehenglieder der behandelten Zehe von Vorteil. Wie bereits erwähnt, dient dabei der als gewindefreie Kreiszylinder ausgebildete distale Endabschnitt der Knochenschraube zum Positionieren der Knochenschraube längs der Zehenlängsachse, was das Fixieren der benachbarten Zehenglieder der behandelten Zehe in der Korrekturstellung erleichtert. Andererseits dient der gewindefreie Mittenabschnitt dazu, den Abstand und somit auch die Druckspannung zwischen den benachbarten Zehengliedern der behandelten Zehe einzustellen.

Um zu erreichen, dass die erfindungsgemäße medizinische Vorrichtung besonders einfach in den Körper des Patienten implantier- und explantierbar ist, weist die Knochenschraube bevorzugt am proximalen Ende ihres proximalen Endabschnittes ferner eine Einrichtung zur Aufnahme eines Werkzeuges zum Eindrehen der Knochenschraube in die jeweiligen Röhrenknochen der ersten und zweiten Zehenglieder des Patienten auf. Dabei ist bevorzugt der proximale Endabschnitt der Knochenschraube wenigstens gleich lang wie der Mittenabschnitt der Knochenschraube ausgeführt, so dass die Knochenschraube, ausgehend von der Zehenspitze, in die Röhrenknochen der jeweiligen Zehenglieder der behandelten Zehe einführbar ist. Im implantierten Zustand kann dabei die Einrichtung zur Aufnahme des Werkzeuges über das zehenspitzenseitige Ende des Röhrenknochens des zehenspitzenseitigen Zehengliedes hervorstehen, was Vorteile für eine leichte Explantation der Knochenschraube nach der Verheilung der behandelten Zehe mit sich bringt.

Im Hinblick auf die Dimensionierung der Knochenschraube der erfindungsgemäßen medizinischen Vorrichtung ist bevorzugt vorgesehen, dass diese einschließlich der Einrichtung zur Aufnahme des Werkzeuges eine Gesamtlänge aufweist, die in einem Bereich zwischen 43 mm bis 45 mm, und vorzugsweise bei 44 mm liegt. Hierbei handelt es sich um Werte, die im Hinblick auf die Größe der Röhrenknochen benachbarter Zehenglieder eines ausgewachsenen Patienten ausgelegt sind.

Andererseits wurde herausgefunden, dass mit der erfindungsgemäßen medizinischen Vorrichtung nicht nur eine optimale Einstellung der auf die benachbarten Zehenglieder auszuübenden leichten Spannung möglich ist, sondern auch ein optimaler Abstand zwischen den Röhrenknochen der benachbarten Zehenglieder leicht einstellbar ist, wenn der Nenndurchmesser des Gewindes des proximalen Gewindeabschnittes in einem Bereich zwischen 2,3 mm bis 2,8 mm, und vorzugsweise bei 2,7 mm liegt, und wenn der Nenndurchmesser des Gewindes des distalen Gewindeabschnittes in einem Bereich zwischen 1,4 mm bis 2,5 mm, und vorzugsweise bei 2,4 mm liegt. Bei diesen für die jeweiligen Nenndurchmesser geltenden Werten kann einerseits ein sicherer Eingriff der jeweiligen Gewinde des distalen bzw. proximalen Gewindeabschnittes mit dem Röhrenknochen des zugehörigen Zehengliedes sichergestellt werden, während gleichzeitig die einfache und problemlose Rückholbarkeit der Knochenschraube ermöglich wird.

Da die Knochenschraube der medizinischen Vorrichtung ausgelegt ist, durch Überbohrung von beispielsweise einem in die jeweiligen Röhrenknochen eingebrachten Bohrloch implantierbar zu sein, ist es bevorzugt, dass die jeweiligen Gewinde des proximalen und distalen Gewindeabschnittes als selbstschneidende Gewinde ausgeführt sind. Da beim Implantieren der Knochenschraube das Gewinde des proximalen Gewindeabschnittes mit dem Röhrenknochen desjenigen Zehengliedes in Eingriff zu bringen ist, durch dessen Röhrenknochen bereits das Gewinde des distalen Gewindeabschnittes der Knochenschraube durchgelaufen ist, ist es im Hinblick auf eine sichere und feste Fixierung der Knochenschraube bevorzugt, dass das Gewinde des proximalen Gewindeabschnittes einen Kerndurchmesser aufweist, der größer als der Kerndurchmesser des Gewindes des distalen Gewindeabschnittes ist.

In einer besonders bevorzugten Realisierung, insbesondere im Hinblick auf die Anwendung der medizinischen Vorrichtung zum Positionieren der Röhrenknochen benachbarter Zehenglieder einer operativ behandelten Zehe in der Korrekturstellung auf der Zehenlängsachse, ist vorgesehen, dass der Kerndurchmesser des Gewindes des proximalen Gewindeabschnittes in einem Bereich zwischen 1,4 mm bis 1,9 mm, und vorzugsweise bei 1,8 mm liegt, und dass der Kerndurchmesser des Gewindes des distalen Gewindeabschnittes in einem Bereich zwischen 0,9 mm bis 1,5 mm, und vorzugsweise bei 1,4 mm liegt. Hierbei handelt es sich um Größenangaben, bei denen eine möglichst optimale Einstellung der behandelten Zehe insbesondere bei einer komplexen Rekonstruktion möglich ist.

Bei einer erfindungsgemäßen Lösung ist der Durchmesser des proximalen Endabschnittes der Knochenschraube größer als der Durchmesser des distalen Endabschnittes der Knochenschraube, wobei in einer bevorzugten Realisierung der Knochenschraube der Durchmesser des proximalen Endabschnittes vorzugsweise in einem Bereich zwischen 1,5 mm bis 1,9 mm und noch bevorzugter bei 1,8 mm liegt, und wobei der Durchmesser des distalen Endabschnittes der Knochenschraube vorzugsweise in einem Bereich zwischen 1,3 mm und 1,6 mm, und noch bevorzugter bei 1,4 mm liegt. Diese Werte sind im Hinblick auf die Größe der zu stabilisierenden Röhrenknochen der Zehenglieder gewählt, wobei als Maß hierzu ein durchschnittlicher (erwachsener) Patient angenommen wurde. Selbstverständlich ist es aber auch denkbar, dass diese Größenwerte abhängig von der Größe des Patienten bzw. der Größe der Röhrenknochen der behandelten Zehe variiert werden.

Um zu erreichen, dass die Knochenschraube der erfindungsgemäßen medizinischen Vorrichtung im implantierten Zustand das zugehörige überbohrte Bohrloch hinreichend abdichtet, ist in einer bevorzugten Weiterentwicklung der erfindungsgemäßen Vorrichtung vorgesehen, dass der Durchmesser des proximalen Endabschnittes der Knochenschraube identisch mit dem Kerndurchmesser des Gewindes des proximalen Endabschnittes ist und vorzugsweise in einem Bereich zwischen 1,5 mm bis 1,9 mm, und noch bevorzugter bei 1,8 mm liegt. Andererseits sollte der Durchmesser des distalen Endabschnittes der Knochenschraube größer als oder gleich groß wie der Kerndurchmesser des Gewindes des distalen Gewindeabschnittes sein und vorzugsweise in einem Bereich zwischen 1,3 mm und 1,6 mm, und noch bevorzugter bei 1,4 mm liegen.

Im Hinblick auf eine möglichst genaue Einstellbarkeit des Abstandes zwischen den in Korrekturstellung vorliegenden Röhrenknochen der benachbarten Zehenglieder ist vorgesehen, dass das Gewinde des proximalen Gewindeabschnittes der Knochenschraube eine Gewindesteigung aufweist, die in einem Bereich zwischen 0,9 bis 1,1, und vorzugsweise bei 1,0 liegt, wobei das Gewinde des distalen Gewindeabschnittes eine Gewindesteigung aufweist, die in einem Bereich zwischen 1,03 bis 1,35 und vorzugsweise in einem Bereich zwischen 1,15 und 1,35, und noch bevorzugter bei 1,25 liegt. Darüber hinaus sollten im Gewinde des proximalen Gewindeabschnittes der Knochenschraube insgesamt etwa 8 oder 9 jeweils gleichweit voneinander beabstandete Gewindegänge vorgesehen sein, wobei im Gewinde des distalen Gewindeabschnittes der Knochenschraube insgesamt etwa 4 jeweils gleichweit voneinander beabstandete Gewindegänge vorgesehen sind. Diese Kennwerte für die jeweiligen Gewinde des proximalen und distalen Gewindeabschnittes ermöglichen eine optimale Einstellung der Spannung zwischen den benachbarten Zehengliedern der behandelten Zehe. Insbesondere kann durch eine leichte Drehung der implantierten Knochenschraube diese Spannung entsprechend erhöht oder verringert werden. Auf diese Weise kann ohne größeres Geschick des Chirurgen die Korrekturstellung der behandelten Zehe sichergestellt werden, wobei gleichzeitig die Zehenglieder im Hinblick auf die Verheilung der behandelten Zehe in einem optimalen Zustand vorliegen.

In der erfindungsgemäßen Vorrichtung beträgt die Länge des proximalen Gewindeabschnittes der Knochenschraube 3 mm bis 5 mm, und vorzugsweise 4 mm, die Länge des distalen Gewindeabschnittes der Knochenschraube 9 mm bis 11 mm, und vorzugsweise 10 mm, die Länge des proximalen Endabschnittes der Knochenschraube 12 mm bis 14 mm, und vorzugsweise 13 mm, und die Länge des distalen Endabschnittes der Knochenschraube 3 mm bis 5 mm, wobei der Abstand zwischen dem proximalen und dem distalen Gewindeabschnitt zwischen 11 mm und 13,5 mm, und vorzugsweise 12 mm beträgt.

Um zu erreichen, dass bei der erfindungsgemäßen medizinischen Vorrichtung die Knochenschraube ohne größeren operativen Eingriff leicht implantierbar und nach zumindest teilweiser Verheilung der behandelten Zehe wieder explantierbar ist, ist in einer bevorzugten Weiterentwicklung der bei der medizinischen Vorrichtung zum Einsatz kommenden Knochenschraube vorgesehen, dass in einer Schnittansicht längs der Knochenschraubenlängsachse das Gewinde des proximalen Gewindeabschnittes eine Vielzahl von Gewindezähnen mit einem ersten Gewindeprofil aufweist, und dass in einer Schnittansicht längs der Knochenschraubenlängsachse das Gewinde des distalen Gewindeabschnittes eine Vielzahl von Gewindezähnen mit einem zum ersten Gewindeprofil identischen zweiten Gewindeprofil aufweist.

Dabei sollte das erste Gewindeprofil eine im wesentlichen dreieckige Formgebung vorzugsweise mit abgerundeten Kanten aufweisen, bei welcher die einschraubseitige Gewindeflanke des Gewindeprofils eines Gewindezahnes mit der Basis des Gewindeprofils einen Winkel von 35° bis 37°, und vorzugsweise 36° einschließt, und bei welcher die dem proximalen Endabschnitt der Knochenschraube zugewandte Gewindeflanke des Gewindeprofils des einen Gewindezahns mit der Basis des Gewindeprofils einen Winkel 51° bis 53 °, und vorzugsweise 52° einschließt. Diese Formgebung der jeweiligen Gewindezähne der Gewinde des proximalen und des distalen Gewindeabschnittes der Knochenschraube ermöglichen ein besonders leichtes Implantieren und Explantieren der Knochenschraube einerseits, und einen sicheren Halt der implantierten Knochenschraube im Inneren der Röhrenknochen der behandelten Zehe andererseits, so dass die Knochenschraube als innere Schiene der Zehe dienen kann. Selbstverständlich sind aber auch andere Gewindeprofile denkbar.

Schließlich ist im Hinblick auf das verwendete Material der bei der medizinischen Vorrichtung zum Einsatz kommenden Gewindeschraube vorgesehen, dass die Knochenschraube aus Titan oder einer Titanlegierung, insbesondere Nitinol besteht. Diese Materialien sind für Implantate aus der Medizintechnik bekannt, und zeichnen sich zum einen durch ihre gute Körperverträglichkeit und zum anderen durch ihre hervorragenden mechanischen Materialeigenschaften aus.

Aus dem vorstehend Gesagten ist es ersichtlich, dass sich die beanspruchte medizinische Vorrichtung mit der Knochenschraube insbesondere zur Verwendung als Einrichtung zum Positionierung der Röhrenknochen von zwei benachbarten Zehengliedern eines Patienten in einer Korrekturstellung auf einen Zehenlängsachse und zum gleichzeitigen Aufbringen einer in Richtung der Zehenlängsachse auf die Röhrenknochen der benachbarten Zehenglieder wirkenden, genau einstellbaren Druckspannung eignet. Diese Verwendung ist derzeit nur aus FR-A-2 808 182 bekannt; vielmehr werden üblicherweise Bohrdrähte, wie beispielsweise Kirschnerdrähte, eingesetzt, um eine innere Schienung der benachbarten Zehenglieder der behandelten Zehe zu ermöglichen.

Aus diesem Grund wird - neben der medizinischen Vorrichtung als solche - auch die Verwendung dieser medizinischen Vorrichtung offenbart, und zwar als Einrichtung zum Positionieren der Röhrenknochen von zwei benachbarten Zehengliedern eines Patienten in einer Korrekturstellung auf der Zehenlängsachse und zum gleichzeitigen Aufbringen einer in Richtung der Zehenlängsachse auf die Röhrenknochen der benachbarten Zehenglieder wirkenden, einstellbaren Druckspannung, vorzugsweise bei der operativen Behandlung von Hammerzehen oder Krallenzehen, bei welcher eine resektionsarthroplastische Operationstechnik zum Einsatz kommt, oder bei der operativen Behandlung eines Hallux Valgus oder eines Hallux Rigidus, bei welcher eine resektionsarthroplastische Operationstechnik zum Einsatz kommt.

Selbstverständlich eignet sich die erfindungsgemäße medizinische Vorrichtung auch für andere orthopädische Anwendungsfälle, bei welchen vorübergehend zwei oder mehrere Knochen einer Zehe in einer Korrekturstellung auf der Zehenlängsachse positioniert und fixiert werden müssen.

Im folgenden werden bevorzugte Realisierungen der erfindungsgemäßen Vorrichtung anhand der beiliegenden Zeichnungen beschrieben. Hierbei handelt es sich um derzeit bevorzugte Ausführungsformen der in der medizinischen Vorrichtung zum Einsatz kommenden Knochenschraube, die jedoch nicht als eine Einschränkung der beanspruchten medizinischen Vorrichtung zu deuten sind.

### Es zeigen:

- Fig. 1a: eine perspektivische Ansicht einer ersten bevorzugten Ausführungsform der bei der erfindungsgemäßen medizinischen Vorrichtung zum Einsatz kommenden zweigängigen Knochenschraube;
- Fig. 1b: eine perspektivische Seitenansicht der Knochenschraube gemäß Fig. 1a;
- Fig. 1c: eine Längsschnittansicht der in Fig. 1b gezeigten Knochenschraube;
- Fig. 2: einen vergrößerten Ausschnitt aus der in Fig. 1c gezeigten Längsschnittdarstellung der bei der medizinischen Vorrichtung gemäß der ersten bevorzugten Ausführungsform zum Einsatz kommenden Knochenschraube, bei welchem der proximale Gewindeabschnitt der Knochenschraube gezeigt ist;
- Fig. 3: einen vergrößerten Ausschnitt aus der in Fig. 1c gezeigten Längsschnittdarstellung der bei der medizinischen Vorrichtung gemäß der ersten bevorzugten Ausführungsform zum Einsatz kommenden Knochenschraube, bei welchem der distale Gewindeabschnitt der Knochenschraube gezeigt ist;
- Fig. 4: eine Draufsicht das proximale Ende des proximalen Endabschnittes der bei der bevorzugten ersten Ausführungsform der medizinischen Vorrichtung zum Einsatz kommenden Knochenschraube;
- Fig. 5a: eine perspektivische Ansicht einer zweiten bevorzugten Ausführungsform der bei der erfindungsgemäßen medizinischen Vorrichtung zum Einsatz kommenden zweigängigen Knochenschraube;
- Fig. 5b: eine perspektivische Seitenansicht der Knochenschraube gemäß Fig. 5a;
- Fig. 5c: eine Längsschnittansicht der in Fig. 5b gezeigten Knochenschraube;
- Fig. 6: einen vergrößerten Ausschnitt aus der in Fig. 5c gezeigten Längsschnittdarstellung der bei der medizinischen Vorrichtung gemäß der zweiten bevorzugten Ausführungsform zum Einsatz kommenden Knochenschraube, bei welchem der proximale Gewindeabschnitt der Knochenschraube gezeigt ist;
- Fig. 7: einen vergrößerten Ausschnitt aus der in Fig. 5c gezeigten Längsschnittdarstellung der bei der medizinischen Vorrichtung gemäß der zweiten bevorzugten Ausführungsform zum Einsatz kommenden Knochenschraube, bei welchem der distale Gewindeabschnitt der Knochenschraube gezeigt ist; und
- Fig. 8: eine Draufsicht auf das distale Ende des distalen Endabschnittes der in Fig. 5b gezeigten Knochenschraube.

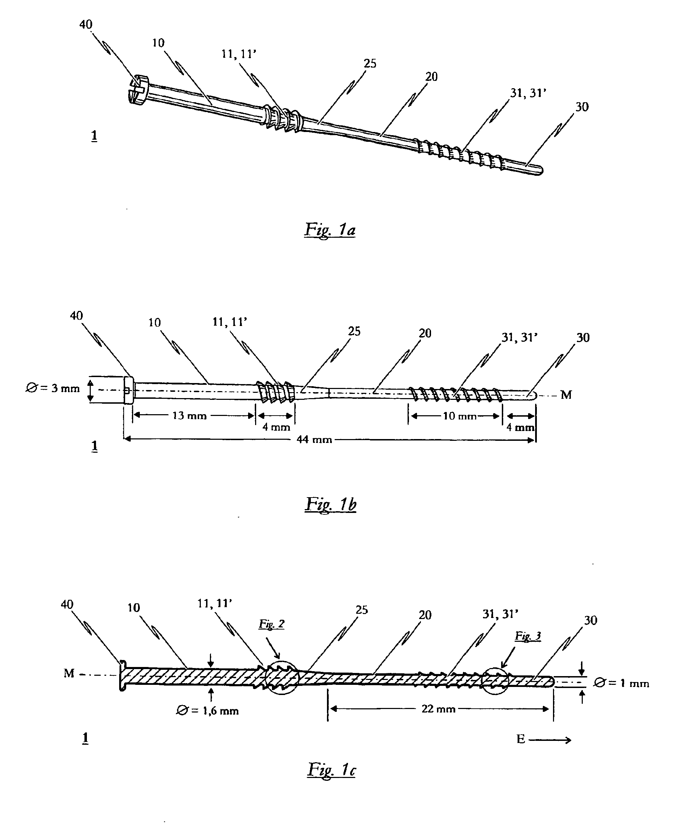

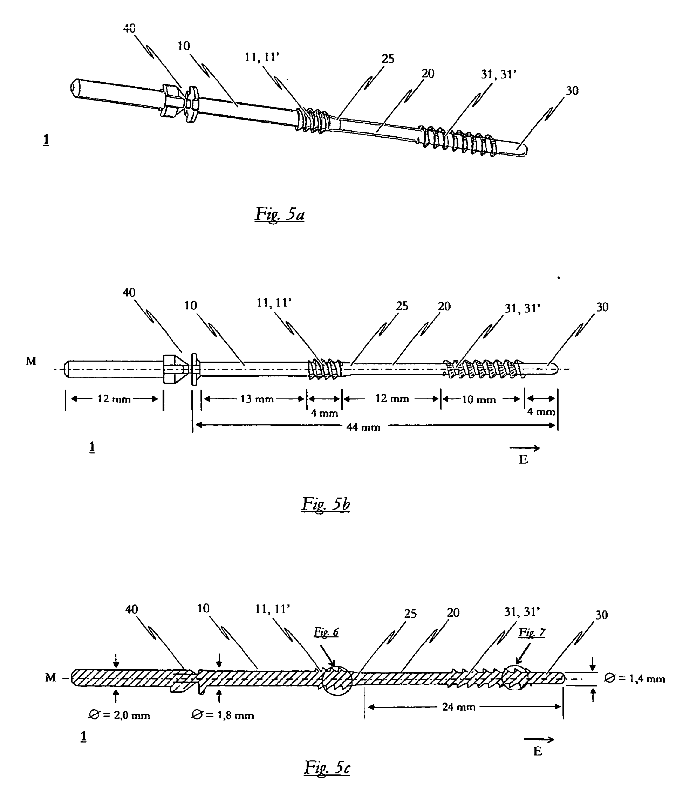

Nachfolgend wird unter Bezugnahme auf die Figuren 1 bis 4 sowie unter Bezugnahme auf die Figuren 5 bis 8 die jeweiligen Knochenschrauben beschrieben, welche bei der medizinischen Vorrichtung gemäß der ersten und der zweiten bevorzugten Ausführungsform zum Einsatz kommen.

Wie dargestellt, weist die medizinische Vorrichtung zum Positionieren der Röhrenknochen benachbarter erster und zweiter Zehenglieder eines Patienten in der Korrekturstellung eine zweigängige Knochenschraube 1 auf, wobei im eingesetzten (implantierten) Zustand der Knochenschraube 1 die Knochenschraubenlängsachse mit der Zehenlängsachse M der behandelten Zehe des Patienten übereinstimmt.

Im einzelnen weist die zweigängige Knochenschraube 1 einen im wesentlichen zylindrischen proximalen Endabschnitt 10, einen im wesentlichen zylindrischen distalen Endabschnitt 30 sowie einen zwischen dem proximalen und distalen Endabschnitt 10, 30 angeordneten und im wesentlichen zylindrischen Mittenabschnitt 20 auf. Diese jeweiligen Abschnitte 10, 20 und 30 sind dabei jeweils als gewindefreie Kreiszylinder ausgebildet.

Des weiteren ist bei der Knochenschraube 1 zwischen dem proximalen Endabschnitt 10 und dem Mittenabschnitt 20 ein proximaler Gewindeabschnitt 11 mit einem ersten Gewinde 11' und zwischen dem distalen Endabschnitt 30 und dem Mittenabschnitt 20 ein distaler Gewindeabschnitt 31 mit einem zweite Gewinde 31 vorgesehen. Anhand der Figuren zu erkennen, dass das erste Gewinde 11' eine Gewindesteigung S1 aufweist, die kleiner als die Gewindesteigung S2 des zweiten Gewindes 31' ist.

Dadurch kann erreicht werden, dass bei Drehung der Knochenschraube 1 um die Knochenschraubenlängsachse M mit den jeweiligen Gewinden 11' und 31' des proximalen und distalen Gewindeabschnittes 11, 31 jeweils Längskräfte erzeugt werden, wobei die mit dem Gewinde 31' des distalen Gewindeabschnitte 31 erzeugte Längskraft größer als die mit dem Gewinde 11' des proximalen Gewindeabschnittes 11 erzeugte Längskraft ist.

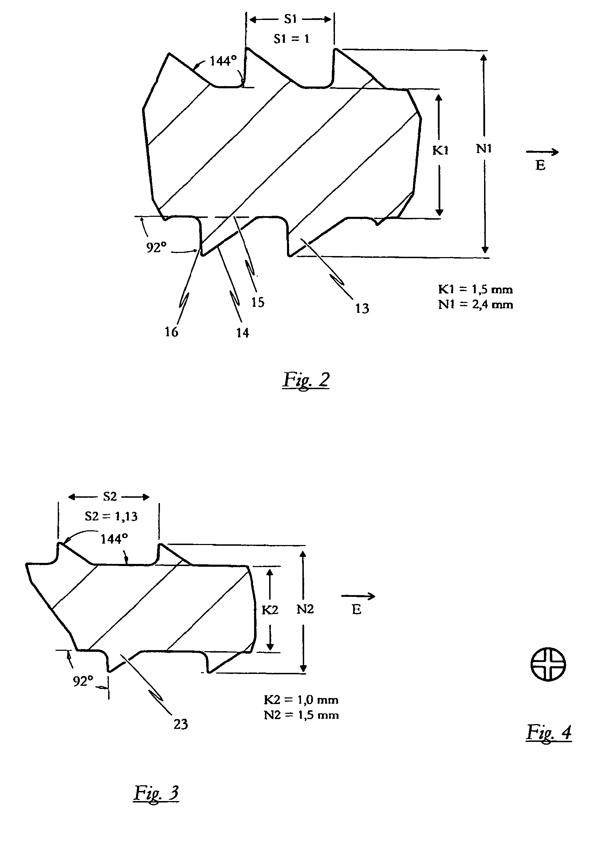

Die Knochenschraube 1, welche bei der medizinischen Vorrichtung gemäß der ersten bevorzugten Ausführungsform zum Einsatz kommt, weist am proximalen Ende des proximalen Endabschnittes 10 ferner eine Einrichtung 40 zur Aufnahme eines Werkzeuges zum Eindrehen der Knochenschraube 1 in die jeweiligen Röhrenknochen der ersten und zweiten Zehenglieder des Patienten auf. Wie es beispielsweise in Fig. 4 zu erkennen ist, ist diese Einrichtung bei der Knochenschraube 1 gemäß der ersten bevorzugten Ausführungsform als Schraubkopf ausgeführt, die zur Aufnahme eines Kreuzschlitzschraubendrehers ausgeführt ist.

Insgesamt liegt die Gesamtlänge der in Fig. 1a bis 1c dargestellten Knochenschraube 1 einschließlich der Einrichtung 40 zur Aufnahme des Werkzeuges bei 40 mm. Ferner ist den Figuren zu entnehmen, dass der Nenndurchmesser bzw. der Kerndurchmesser des im proximalen Gewindeabschnitt 11 vorgesehenen ersten Gewindes 11' bei 2,4 mm bzw. 1,5 mm liegt. Andererseits beträgt der Nenndurchmesser des zweiten Gewindes 31' im distalen Gewindeabschnitt 31 1,6 mm, während der Kerndurchmesser hiervon bei 1,5 mm liegt.

Ferner ist bei der Knochenschraube 1 gemäß Fig. 1a bis 1c der proximale Endabschnitt 10 mit einem Durchmesser von 1,6 mm und der distale Endabschnitt 30 mit einem Durchmesser von 1,5 mm ausgeführt, während der proximale Endabschnitt 10 eine Länge von 13 mm und der distale Endabschnitt 30 eine Länge von 4 mm aufweist. Der proximale Gewindeabschnitt 11 und der distale Gewindeabschnitt 31 weisen jeweils eine Länge von 4 bzw. 10 mm auf.

Nachfolgend werden unter Bezugnahme auf Fig. 2 und Fig. 3 die Gewindekenngrößen der Gewinde 11' und 31' des proximalen und distalen Gewindeabschnittes 11 und 31 der in Fig. 1 dargestellten Knochenschraube beschrieben.

Der Zusammenschau der Fig. 2 und der Fig. 3 zeigt, dass in einer Schnittansicht längs der Knochenschraublängsachse M das Gewinde 11' das proximalen Gewindeabschnittes 11 Gewindezähne 13 mit einem ersten Gewindeprofil aufweist, und dass in einer Schnittansicht längs der Knochenschraublängsachse M das Gewinde 31' des distalen Gewindeabschnittes 31 Gewindezähne 33 mit einem zum ersten Gewindeprofil identischen zweiten Gewindeprofil aufweist. Im einzelnen sind beide Gewindeprofile im wesentlichen dreieckig mit abgerundeten Kannten ausgeführt, wobei die einschraubseitige Gewindeflanke 14 des Gewindeprofils eines Gewindezahnes 13, 33 mit der Basis 15 des Gewindeprofils einen Winkel von etwa 36° einschließt, und wobei die dem proximalen Endabschnitt 10 der Knochenschraube 1 zugewandte Gewindeflanke 16 des Gewindeprofils des einen Gewindezahnes 13, 33 mit der Basis 15 des Gewindeprofils einen Winkel von etwa 52° einschließt.

Die in Fig. 5 bis Fig. 8 dargestellte Knochenschraube 1 gemäß der zweiten bevorzugten Ausführungsform der erfindungsgemäßen medizinischen Vorrichtung ist im Prinzip identisch mit der unter Bezugnahme auf Fig. 1 bis Fig. 4 beschriebenen ersten Ausführungsform. Allerdings liegen in der jeweiligen Dimensionierung der einzelnen Abschnitte der Knochenschrauben gewisse Unterschiede vor, wie es anhand der in der nachfolgenden Tabelle 1 aufgeführten Kenngrößen der Knochenschraube gemäß der ersten und zweiten bevorzugten Ausführungsform zu erkennen ist.

**Tabelle 1**

| **Kenngröße/Dimensionierung** | **Erste Ausführungsform** | **Zweite Ausführungsform** | **Toleranz** |
|---|---|---|---|
| Nenndurchmesser N1 des ersten Gewindes 11' | 2,4 mm | 2,7 mm | ± 0,10 mm |
| Nenndurchmesser N2 des zweiten Gewindes 31' | 1,5 mm | 2,4 mm | ± 0,10 mm |
| Kerndurchmesser K1 des ersten Gewindes 11' | 1,5 mm | 1,8 mm | ± 0,10 mm |
| Kerndurchmesser K2 des zweiten Gewindes 31' | 1,0 mm | 1,4 mm | ± 0,10 mm |
| Durchmesser D1 des proximalen Endabschnittes 10 | 1,6 mm | 1,8 mm | ± 0,10 mm |
| Durchmesser D2 des distalen Endabschnittes 30 | 1,0 mm | 1,4 mm | ± 0,10 mm |
| Länge des proximalen Endabschnittes 10 | 13,0 mm | 13,0 mm | ± 0,25 mm |
| Länge des distalen Endabschnittes 30 | 4,0 mm | 4,0 mm | ± 0,10 mm |
| Länge des proximalen Gewindeabschnittes 11 | 4,0 mm | 4,0 mm | ± 0,10 mm |
| Länge des distalen Gewindeabschnittes 31 | 10,0 mm | 10,0 mm | ± 0,25 mm |
| Länge des Mittenabschnittes 20 mit Übergangabschnitt 25 | 12,0 mm | 12,0 mm | ± 0,25 mm |
| Länge des Übergangabschnitt 25 | 2,0 mm | 2,0 mm | ± 0,10 mm |
| Gesamtlänge der Schraube 1 mit Einrichtung 40 | 44,0 mm | 44,0 mm | ± 0,50 mm |

Des weiteren unterscheidet sich die Knochenschraube 1 gemäß der zweiten Ausführungsform durch die Ausführung der Einrichtung 40 zur Aufnahme eines Werkzeuges zum Eindrehen der Knochenschraube 1 in die jeweiligen Röhrenknochen der ersten und zweiten Zehenglieder des Patienten. Wie in den Figuren 5a bis 5c dargestellt, ist bei der zweiten bevorzugten Ausführungsform diese Einrichtung 40 in Gestalt eines an dem Schraubkopf befestigten Dornes ausgeführt, der in Eingriff mit dem Werkzeug zum Eindrehen der Knochenschraube 1 bringbar ist.

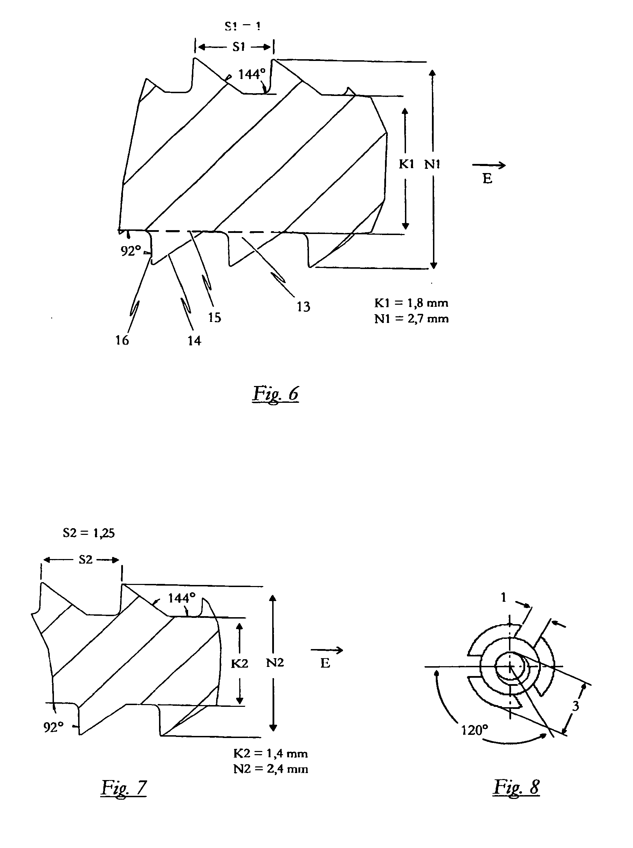

In Fig. 6 und Fig. 7 sind die jeweiligen Gewinde des proximalen und distalen Gewindeabschnittes der in Fig. 5a bis 5c gezeigten Knochenschraube 1 gemäß der zweiten Ausführungsform dargestellt. Der Vergleich der Fig. 6 und der Fig. 7 mit der Fig. 2 und der Fig. 3 zeigt, dass bei beiden Ausführungsformen die Gewindeprofile identisch sind.

In Fig. 8 ist eine Draufsicht auf das distale Ende des distalen Endabschnittes der in Fig. 5a bis 5c gezeigten Knochenschraube 1 gezeigt.

Die Erfindung ist nicht auf die in den Figuren gezeigten und speziellen Ausführungsformen beschränkt. Insbesondere können die angegebenen Dimensionierungen und Kenngrößen in Abhängigkeit des jeweiligen Einzelfalls abgeändert werden so weit diese den Ansprüchen unterzuordnen sind.

### Bezugszeichenliste

- 1: Knochenschraube
- 10: proximaler Endabschnitt
- 11: proximaler Gewindeabschnitt
- 11': erstes Gewinde/Gewindes des proximalen Gewindeabschnittes
- 13: Gewindezahn des ersten Gewindes
- 14: einschraubseitige Gewindeflanke
- 15: Gewindezahn-Basis
- 16: schraubenkopfseitige Gewindeflanke
- 20: Mittenabschnitt
- 25: Übergangsabschnitt
- 30: distaler Endabschnitt
- 31: distaler Gewindeabschnitt
- 31': zweites Gewinde/Gewindes des distalen Gewindeabschnittes
- 33: Gewindezahn des zweiten Gewindes
- 40: Einrichtung zur Aufnahme eines Werkzeuges/Schraubkopf

- D1: Durchmesser des proximalen Endabschnittes
- D2: Durchmesser des distalen Endabschnittes
- E: Einschraubrichtung
- K1: Kerndurchmesser des ersten Gewindes
- K2: Kerndurchmesser des zweiten Gewindes
- M: Zehenlängsachse
- N1: Nenndurchmesser des ersten Gewindes
- N2: Nenndurchmesser des zweiten Gewindes

## Patentansprüche

1. Vorrichtung zum Positionieren der Röhrenknochen benachbarter erster und zweiter Zehenglieder eines Patienten auf der Zehenlängsachse (M), wobei die Vorrichtung eine zweigängige Knochenschraube (1) mit einem im wesentlichen zylindrischen proximalen Endabschnitt (10), einem im wesentlichen zylindrischen distalen Endabschnitt (30) und einem zwischen dem proximalen und distalen Endabschnitt (10, 30) angeordneten und im wesentlichen zylindrischen Mittenabschnitt (20) aufweist, wobei zwischen dem proximalen Endabschnitt (10) und dem Mittenabschnitt (20) der Knochenschraube (1) ein proximaler Gewindeabschnitt (11) mit einem ersten Gewinde (11') und zwischen dem distalen Endabschnitt (30) und dem Mittenabschnitt (20) der Knochenschraube (10) ein distaler Gewindeabschnitt (31) mit einem zweiten Gewinde (31') vorgesehen sind, wobei
- das erste Gewinde (11') eine Gewindesteigung (S1) aufweist, die kleiner als die Gewindesteigung (S2) des zweiten Gewindes (31') ist;
- der Nenndurchmesser (N1) des ersten Gewindes (11') größer als der Durchmesser (D1) des proximalen Endabschnittes (10) ist, und wobei der Nenndurchmesser (N2) des zweiten Gewindes (31') größer als der Durchmesser (D2) des distalen Endabschnittes (30) und gleich groß wie oder kleiner als der Durchmesser (D1) des proximalen Endabschnittes (10) ist, und wobei
- der proximale Endabschnitt (10) der Knochenschraube (1) wenigstens gleich lang wie der Mittenabschnitt (20) ist,
**dadurch gekennzeichnet, dass**
das Gewinde (11') des proximalen Gewindeabschnittes (11) der Knochenschraube (1) eine Gewindesteigung aufweist, die in einem Bereich zwischen 0,9 bis 1,1 und vorzugsweise bei 1,0 liegt, und dass das Gewinde (31') des distalen Gewindeabschnittes (31) eine Gewindesteigung aufweist, die in einem Bereich zwischen 1,03 bis 1,35 und vorzugsweise in einem Bereich zwischen 1,15 und 1,35 und noch bevorzugter bei 1,25 liegt; und dass
der proximale Gewindeabschnitt (11) der Knochenschraube (1) eine Länge von 3 bis 5 mm und vorzugsweise 4 mm, der distale Gewindeabschnitt (31) der Knochenschraube (1) eine Länge von 9 bis 11 mm und vorzugsweise 10 mm, der proximale Endabschnitt (10) der Knochenschraube (1) eine Länge von 12 bis 14 mm und vorzugsweise 13 mm, und der distale Endabschnitt (30) der Knochenschraube (1) eine Länge von 3 bis 5 mm und vorzugsweise 4 mm aufweist, und dass der Abstand zwischen dem proximalen und dem distalen Gewindeabschnitt (11, 31) zwischen 11 und 13,5 mm und vorzugsweise 12 mm beträgt.

2. Vorrichtung nach Anspruch 1, wobei
der proximale Endabschnitt (10), der distale Endabschnitt (30) und der Mittenabschnitt (20) zwischen dem proximalen und distalen Endabschnitt (10, 30) der Knochenschraube (1) jeweils als gewindefreie Kreiszylinder ausgebildet sind.

3. Vorrichtung nach Anspruch 1 oder 2, wobei
am proximalen Ende des proximalen Endabschnittes (10) der Knochenschraube (1) ferner eine Einrichtung (40) zur Aufnahme eines Werkzeuges zum Eindrehen der Knochenschraube (1) in die jeweiligen Röhrenknochen der ersten und zweiten Zehenglieder des Patienten aufweist.

4. Vorrichtung nach Anspruch 3, wobei
die Gesamtlänge der Knochenschraube (1) einschließlich der Einrichtung (40) zur Aufnahme des Werkzeuges in einem Bereich zwischen 43 bis 45 mm und vorzugsweise bei 44 mm liegt.

5. Vorrichtung nach einem der vorhergehenden Ansprüche, wobei
der Nenndurchmesser (N1) des Gewindes (11') des proximalen Gewindeabschnittes (11) in einem Bereich zwischen 2,3 bis 2,8 mm und vorzugsweise bei 2,7 mm liegt, und/oder wobei der Nenndurchmesser (N2) des Gewindes (31') des distalen Gewindeabschnittes (31) in einem Bereich zwischen 1,4 bis 2,5 mm und vorzugsweise bei 2,4 mm liegt.

6. Vorrichtung nach einem der vorhergehenden Ansprüche, wobei
das Gewinde (11') des proximalen Gewindeabschnittes (11) der Knochenschraube (1) einen Kerndurchmesser (K1) aufweist, der größer als der Kerndurchmesser (K2) des Gewindes (31') des distalen Gewindeabschnittes (31) der Knochenschraube (1) ist.

7. Vorrichtung nach Anspruch 6, wobei
der Kerndurchmesser (K1) des Gewindes (11') des proximalen Gewindeabschnittes (11) in einem Bereich zwischen 1,4 bis 1,9 mm, und vorzugsweise bei 1,8 mm liegt, und/oder wobei der Kerndurchmesser (K2) des Gewindes (31') des distalen Gewindeabschnittes (31) in einem Bereich zwischen 0,9 bis 1,5 mm, und vorzugsweise bei 1,4 mm liegt.

8. Vorrichtung nach einem der vorhergehenden Ansprüche, wobei
der Durchmesser (D1) des proximalen Endabschnittes (10) der Knochenschraube (1) größer als der Durchmesser (D2) des distalen Endabschnittes (30) der Knochenschraube (1) ist, und wobei der Durchmesser (D1) des proximalen Endabschnittes (10) vorzugsweise in einem Bereich zwischen 1,5 bis 1,9 mm und noch bevorzugter bei 1,8 mm liegt, und wobei der Durchmesser (D2) des distalen Endabschnittes (30) der Knochenschraube (1) vorzugsweise in einem Bereich zwischen 1,3 und 1,6 mm und noch bevorzugter bei 1,4 mm liegt.

9. Vorrichtung nach einem der vorhergehenden Ansprüche, wobei
der Durchmesser (D1) des proximalen Endabschnittes (10) der Knochenschraube (1) identisch mit dem Kerndurchmesser (K1) des Gewindes (11') des proximalen Gewindeabschnittes (11) der Knochenschraube ist und vorzugsweise in einem Bereich zwischen 1,5 bis 1,9 mm und noch bevorzugter bei 1,8 mm liegt.

10. Vorrichtung nach einem der vorhergehenden Ansprüche, wobei
der Durchmesser (D2) des distalen Endabschnittes (30) der Knochenschraube (1) größer als oder gleich groß wie der Kerndurchmesser (K2) des Gewindes (31') des distalen Gewindeabschnittes (31) der Knochenschraube (1) ist und vorzugsweise in einem Bereich zwischen 1,3 und 1,6 mm, und noch bevorzugter bei 1,4 mm liegt.

11. Vorrichtung nach einem der vorhergehenden Ansprüche, wobei
im Gewinde (11') des proximalen Gewindeabschnittes (11) der Knochenschraube (1) insgesamt 8 oder 9 jeweils gleich weit voneinander beabstandete Gewindegänge vorgesehen sind, und wobei im Gewinde (31') des distalen Gewindeabschnittes (31) der Knochenschraube (1) insgesamt vier jeweils gleich weit voneinander beabstandete Gewindegänge vorgesehen sind.

12. Vorrichtung nach einem der vorhergehenden Ansprüche, wobei
zwischen dem Mittenabschnitt (20) der Knochenschraube (1) und dem proximalen Gewindeabschnitt (11) der Knochenschraube (1) ein Übergangsabschnitt (25) mit einer sich in Einschraubrichtung (E) der Knochenschraube (1) verjüngenden Querschnittsformgebung vorgesehen ist.

13. Vorrichtung nach einem der vorhergehenden Ansprüche, wobei
in einer Schnittansicht längs der Knochenschraubenlängsachse (M) das Gewinde (11') des proximalen Gewindeabschnittes (11) Gewindezähne (13) mit einem ersten Gewindeprofil aufweist, und wobei in einer Schnittansicht längs der Knochenschraubenlängsachse (M) das Gewinde (31') des distalen Gewindeabschnittes (31) Gewindezähne (33) mit einem zum ersten Gewindeprofil identischen zweiten Gewindeprofil aufweist.

14. Vorrichtung nach Anspruch 13, wobei
das erste Gewindeprofil eine im wesentlichen dreieckige Formgebung vorzugsweise mit abgerundeten Kanten aufweist, bei welcher die einschraubseitige Gewindeflanke (14) des Gewindeprofils eines Gewindezahnes (13) mit der Basis (15) des Gewindeprofils einen Winkel von 35 bis 37° und vorzugsweise 36° einschließt, und bei welcher die dem proximalen Endabschnitt (10) der Knochenschraube (1) zugewandte Gewindeflanke (16) des Gewindeprofils des einen Gewindezahns (13) mit der Basis (15) des Gewindeprofils einen Winkel von 51 bis 53° und vorzugsweise 52° einschließt.

15. Vorrichtung nach einem der vorhergehenden Ansprüche, wobei
die Knochenschraube (1) aus Titan oder einer Titanlegierung, insbesondere Nitinol besteht.

## Claims

1. A device for positioning tubular bones of a patient's neighboring first and second phalanges on the longitudinal axis (M) of the toe, wherein the device comprises a double-threaded bone screw (1) having a substantially cylindrical proximal end section (10), a substantially cylindrical distal end section (30) and a substantially cylindrical central section (20) arranged between the proximal and distal end sections (10, 30), wherein a proximal thread section (11) having a first thread (11') is provided between the proximal end section (10) and the central section (20) of the bone screw (1) and a distal thread section (31) having a second thread (31') is provided between the distal end section (30) and the central section (20) of the bone screw (10), wherein
- the first thread (11') has a pitch (S1) which is smaller than the pitch (S2) of the second thread (31');.
- the nominal diameter (N1) of the first thread (11') is larger than the diameter (D1) of the proximal end section (10), and wherein the nominal diameter (N2) of the second thread (31') is larger than the diameter (D2) of the distal end section (30) and just as large or smaller than the diameter (D1) of the proximal end section (10), and wherein the proximal end section (10) of the bone screw (1) is at least as long as the central section (20),
**characterized in that**
the thread (11') of the proximal thread section (11) of the bone screw (1) exhibits a pitch in the range of between 0.9 and 1.1, and preferably 1.0, and the thread (31') of the distal thread section (31) exhibits a pitch in the range of between 1.03 and 1.35, and preferably in the range of between 1.15 and 1.35, and is more preferable at 1.25; and that
the proximal thread section (11) of the bone screw (1) has a length of from 3 to 5 mm, and preferably 4 mm, the distal thread section (31) of the bone screw (1) has a length of from 9 to 11 mm, and preferably 10 mm, the proximal end section (10) of the bone screw (1) has a length of from 12 to 14 mm, and preferably 13 mm, and the distal end section (30) of the bone screw (1) has a length of from 3 to 5 mm, and preferably 4 mm, and that the distance between the proximal and the distal thread section (11, 31) is between 11 and 13.5 mm, and preferably 12 mm.

2. The device according to claim 1, wherein
the proximal end section (10), the distal end section (30) and the central section (20) between the proximal and distal end sections (10, 30) of the bone screw (1) are each respectively configured as unthreaded circular cylinders.

3. The device according to claim 1 or 2, wherein
a mechanism (40) is further provided on the proximal end of the proximal end section (10) of the bone screw (1) to receive a tool for screwing the bone screw (1) into the respective tubular bones of the patient's first and second phalanges.

4. The device according to claim 3, wherein
the full length of the bone screw (1) including the mechanism (40) for receiving the tool measures within a range of between 43 and 45 mm, and is preferably 44 mm.

5. The device according to any one of the preceding claims, wherein
the nominal diameter (N1) of the thread (11') of the proximal thread section (11) is in a range of between 2.3 and 2.8 mm, and is preferably 2.7 mm, and/or wherein the nominal diameter (N2) of the thread (31') of the distal thread section (31) is in a range of between 1.4 and 2.5 mm, and is preferably 2.4 mm.

6. The device according to any one of the preceding claims, wherein
the thread (11') of the proximal thread section (11) of the bone screw (1) exhibits a core diameter (K1) which is larger than the core diameter (K2) of the thread (31') of the distal thread section (31) of the bone screw (1).

7. The device according to claim 6, wherein
the core diameter (K1) of the thread (11') of the proximal thread section (11) is in a range of between 1.4 and 1.9 mm, and is preferably 1.8 mm, and/or wherein the core diameter (K2) of the thread (31') of the distal thread section (31) is in a range of between 0.9 and 1.5 mm, and is preferably 1.4 mm.

8. The device according to any one of the preceding claims, wherein
the diameter (D1) of the proximal end section (10) of the bone screw (1) is larger than the diameter (D2) of the distal end section (30) of the bone screw (1), and wherein the diameter (D1) of the proximal end section (10) is preferably in a range of between 1.5 and 1.9 mm, and is more preferable at 1.8 mm, and wherein the diameter (D2) of the distal end section (30) of the bone screw (1) is preferably in a range of between 1.3 and 1.6 mm, and is more preferable at 1.4 mm.

9. The device according to any one of the preceding claims, wherein
the diameter (D1) of the proximal end section (10) of the bone screw (1) is identical to the core diameter (K1) of the thread (11') of the proximal thread section (11) of the bone screw and preferably in a range of between 1.5 and 1.9 mm, and is more preferable at 1.8 mm.

10. The device according to any one of the preceding claims, wherein
the diameter (D2) of the distal end section (30) of the bone screw (1) is larger or just as large as the core diameter (K2) of the thread (31') of the distal thread section (31) of the bone screw (1) and preferably in a range of between 1.3 and 1.6 mm, and is more preferable at 1.4 mm

11. The device according to any one of the preceding claims, wherein
a total of 8 or 9 thread turns of respectively equal distance from one another are provided in the thread (11') of the proximal thread section (11) of the bone screw (1), and wherein a total of four thread turns of respectively equal distance from one another are provided in the thread (31') of the distal thread section (31) of the bone screw (1).

12. The device according to any one of the preceding claims, wherein
a transitional section (25) having a cross-sectional tapering shape in the screwing direction (E) of the bone screw (1) is provided between the central section (20) of the bone screw (1) and the proximal thread section (11) of the bone screw (1).

13. The device according to any one of the preceding claims, wherein
the thread (11') of the proximal thread section (11) exhibits threaded teeth (13) having a first thread profile when viewed sectionally along the bone screw longitudinal axis (M), and wherein the thread (31') of the distal thread section (31) exhibits threaded teeth (33) having a second thread profile identical to the first thread profile when viewed sectionally along the bone screw longitudinal axis (M).

14. The device according to claim 13, wherein
the first thread profile exhibits a substantially triangular shape, preferably with rounded edges, in which the screwing-side thread flank (14) of the thread profile of a threaded tooth (13) forms an angle of from 35 to 37°, and preferably 36°, with the base (15) of the thread profile, and in which the thread flank (16) of the thread profile of said one threaded tooth (13) facing the proximal end section (10) of the bone screw (1) forms an angle of from 51 to 53°, and preferably 52°, with the base (15) of the thread profile.

15. The device according to any one of the preceding claims, wherein
the bone screw (1) is made from titanium or a titanium alloy, in particular Nitinol.

## Revendications

1. Dispositif pour le positionnement des os tubulaires de la première et de la seconde phalange voisines d'un orteil d'un patient sur l'axe longitudinal (M) de l'orteil, ledit dispositif comprenant une vis à os (1) à deux filets avec un tronçon terminal proximal sensiblement cylindrique (10), un tronçon terminal distal sensiblement cylindrique (30) et un tronçon médian sensiblement cylindrique (20) agencé entre le tronçon terminal proximal et le tronçon terminal distal (10, 30), dans lequel entre le tronçon terminal proximal (10) et le tronçon médian (20) de la vis à os (1) il est prévu un tronçon fileté proximal (11) avec un premier filetage (11') et entre le tronçon terminal distal (30) et le tronçon médian (20) de la vis à os (10) il est prévu un tronçon fileté distal (31) avec un second filetage (31'), dans lequel
- le premier filetage (11') présente un premier pas de filetage (S1) qui est inférieur au pas (S2) du second filetage (31') ;
- le diamètre nominal (N1) du premier filetage (11') est supérieur au diamètre (D1) du tronçon terminal proximal (10) et le diamètre nominal (N2) du second filetage (31') est supérieur au diamètre (D2) du tronçon terminal distal (30) et est égal ou inférieur au diamètre (D1) du tronçon terminal proximal (10), et dans lequel
- le tronçon terminal proximal (10) de la vis à os (1) est au moins aussi long que le tronçon médian (20),
**caractérisé en ce que**
le filetage (11') du tronçon fileté proximal (11) de la vis à os (1) présente un pas de filetage qui est situé dans une plage entre 0,9 et 1,1 et de préférence environ 1,0, et **en ce que** le filetage (31') du tronçon fileté distal (31) présente un pas de filetage qui est situé dans une plage entre 1,03 et 1,35 et de préférence dans une plage entre 1,15 et 1,35, et de façon encore plus préférée environ 1,25, et **en ce que**
le tronçon fileté proximal (11) de la vis à os (1) a une longueur de 3 à 5 mm et de préférence 4 mm, le tronçon fileté distal (31) de la vis à os (1) a une longueur de 9 à 11 mm et de préférence 10 mm, le tronçon terminal proximal (10) de la vis à os (1) a une longueur de 12 à 14 mm et de préférence 13 mm, et le tronçon terminal distal (30) de la vis à os (1) a une longueur de 3 à 5 mm et de préférence 4 mm, et **en ce que** la distance entre le tronçon fileté proximal et le tronçon fileté distal (11, 31) est entre 11 et 13,5 mm et de préférence 12 mm.

2. Dispositif selon la revendication 1, dans lequel
le tronçon terminal proximal (10), le tronçon terminal distal (30) et le tronçon médian (20) entre le tronçon terminal proximal et le tronçon terminal distal (10, 30) de la vis à os (1) sont réalisés respectivement sous forme de cylindres droits dépourvus de filetage.

3. Dispositif selon la revendication 1 ou 2, dans lequel
à l'extrémité proximale du tronçon terminal proximal (10) de la vis à os (1) il est en outre prévu un moyen (40) pour la réception d'un outil pour visser la vis à os (1) dans les os tubulaires respectifs de la première et de la seconde phalange d'orteil du patient.

4. Dispositif selon la revendication 3, dans lequel
la longueur totale de la vis à os (1) y compris le moyen (40) pour la réception de l'outil, est dans une plage entre 43 et 45 mm et de préférence environ 44 mm.

5. Dispositif selon l'une des revendications précédentes, dans lequel
le diamètre nominal (N1) du filetage (11') du tronçon fileté proximal (11) est dans une plage entre 2,3 et 2,8 mm et de préférence environ 2,7 mm, et/ou dans lequel le diamètre nominal (N2) du filetage (31') du tronçon fileté distal (31) est dans une plage entre 1,4 et 2,5 mm et de préférence environ 2,4 mm.

6. Dispositif selon l'une des revendications précédentes, dans lequel
le filetage (11') du tronçon fileté proximal (11) de la vis à os (1) a un diamètre à fond de filet (K1) qui est supérieur au diamètre à fond de filet (K2) du filetage (31') du tronçon fileté distal (31) de la vis à os (1).

7. Dispositif selon la revendication 6, dans lequel
le diamètre à fond de filet (K1) du filetage (11') du tronçon fileté proximal (11) est dans une plage entre 1,4 et 1,9 mm, et de préférence environ 1,8 mm, et/ou dans lequel le diamètre à fond de filet (K2) du filetage (31') du tronçon fileté distal (31) est dans une plage entre 0,9 et 1,5 mm, et de préférence environ 1,4 mm.

8. Dispositif selon l'une des revendications précédentes, dans lequel
le diamètre (D1) du tronçon terminal proximal (10) de la vis à os (1) est supérieur au diamètre (D2) du tronçon terminal distal (30) de la vis à os (1), et dans lequel le diamètre (D1) du tronçon terminal proximal (10) est de préférence dans une plage entre 1,5 et 1,9 mm et de façon encore plus préférée environ 1,8 mm, et dans lequel le diamètre (D2) du tronçon terminal distal (30) de la vis à os (1) est de préférence dans une plage entre 1,3 et 1,6 mm et de façon encore plus préférée environ 1,4 mm.

9. Dispositif selon l'une des revendications précédentes, dans lequel
le diamètre (D1) du tronçon terminal proximal (10) de la vis à os (1) est identique avec le diamètre à fond de filet (K1) du filetage (11') du tronçon fileté proximal (11) de la vis à os et est de préférence dans une plage entre 1,5 et 1,9 mm, et de façon encore plus préférée environ 1,8 mm.

10. Dispositif selon l'une des revendications précédentes, dans lequel
le diamètre (D2) du tronçon terminal distal (30) de la vis à os (1) est supérieur ou égal au diamètre à fond de filet (K2) du filetage (31') du tronçon fileté distal (31) de la vis à os (1) et est de préférence dans une plage entre 1,3 et 1,6 mm, et de façon encore plus préférée environ 1,4 mm.

11. Dispositif selon l'une des revendications précédentes, dans lequel
dans le filetage (11') du tronçon fileté proximal (11) de la vis à os (1) il est prévu au total 8 ou 9 spires de filetage respectivement écartées à la même distance les unes des autres, et dans le filetage (31') du tronçon fileté distal (31) de la vis à os (1) il est prévu au total quatre spires de filetage respectivement écartées à la même distance les unes des autres.

12. Dispositif selon l'une des revendications précédentes, dans lequel
un tronçon de transition (25), avec une conformation de section transversale qui va en se rétrécissant dans la direction de vissage (E) de la vis à os (1), est prévu entre le tronçon médian (20) de la vis à os (1) et le tronçon fileté proximal (11) de la vis à os (1).

13. Dispositif selon l'une des revendications précédentes, dans lequel
dans une vue en coupe le long de l'axe longitudinal de la vis à os (M), le filetage (11') du tronçon fileté proximal (11) présente des dents de filetage (13) avec un premier profil de filetage, et dans une vue en coupe le long de l'axe longitudinal de la vis à os (M), le filetage (31') du tronçon fileté distal (31) présente des dents de filetage (33) avec un second profil de filetage identique au premier profil de filetage.

14. Dispositif selon la revendication 13, dans lequel
le premier profil de filetage présente une conformation sensiblement triangulaire, de préférence avec des arêtes arrondies, dans laquelle le flanc de filetage (14) du profil de filetage situé côté vissage, d'une dent de filetage (13) définit avec la base (15) du profil de filetage un angle de 35 à 37° et de préférence 36°, et dans lequel le flanc de filetage (16) du profil de filetage tourné vers le tronçon terminal proximal (10) de la vis à os (1), de la dent de filetage (13) concernée, définit avec la base (15) du profil de filetage un angle de 51 à 53° et de préférence 52°.

15. Dispositif selon l'une des revendications précédentes, dans lequel
la vis à os (1) est formée en titane ou en un alliage de titane, en particulier connu sous la dénomination Nitinol.
